# EUROPEAN PATENT SPECIFICATION

(11) **EP 1 619 996 B1**
(45) Date of publication and mention of the grant of the patent: **05.12.2012**
(21) Application number: 04750825.4
(22) Date of filing: 26.04.2004
(51) Int. Cl.: A61M 31/00, A61B 18/14, A61M 37/00

(54) **STEERABLE KINK-RESISTANT SHEATH**
LENKBARE KNICKFESTE HÜLSE
GAINE DIRIGEABLE RESISTANT AU PLIAGE

(30) Priority: 25.04.2003 US 465310 P
(43) Date of publication of application: 01.02.2006
(60) Divisional application: 11181317.6
(73) Proprietor: APPLIED MEDICAL RESOURCES CORPORATION, Rancho Santa Margarita, CA 92688 (US)
(72) Inventor: HART, Charles, C., Summerville, SC (US); BRUSTAD, John, R., Dana Point, CA 92629 (US); HILAL, Nabil, Laguna Niguel, CA 92677 (US); KAHLE, Henry, Trabuco Canyon, CA 92679 (US); GADBERRY, Donald L., San Clemente, CA 92672 (US)
(74) Representative: Fitchett, Stuart Paul
(86) International application number: PCT/US2004/013118
(87) International publication number: WO 2004/096015

(56) References cited:
- EP-A- 0 605 796
- US-A- 2 688 329
- US-A- 3 503 385
- US-A- 5 827 278
- US-A- 5 865 800
- US-A- 6 146 355
- US-B1- 6 544 215

## Description

### BACKGROUND

The present invention generally relates to surgical access devices and, more specifically, to kink resistant sheaths having steerable sections that enable the sheaths to access hard-to-reach body cavities and conduits.

Sheaths and catheters have long been used to access body conduits such as the arterial and venous branches of the vascular system, urinary tract, body cavities such as the thorax and abdomen, and hollow viscous organs such as the stomach, intestines and urinary bladder. More specifically, sheaths and catheters have been used for fluid delivery, fluid recovery, implant delivery and for providing an access pathway for an instrument such as an endoscope. However, many endoscopes, for example, are flexible enough to bend but are not steerable or deflectable in a controlled and/or dynamic manner.

For some instruments, steering has been achieved, for example, by "pre-bending" the distal tip of a surgical device before insertion and then rotating the device once it has been inserted and has reached a branch artery inside the body. If the angle of the bend has to be adjusted, then the device may have to be removed, re-bent and reinserted. This results in greater time spent in the body and thereby increase surgery time. Furthermore, since these sheaths and catheters need to navigate many hard-to-reach areas, it follows that they should be as stiff and yet as flexible as possible. It is also useful that the sheaths and catheters are constructed with thin walls to minimize the diameter of the device and to maximize the radii of the internal lumen.

In trying to balance the flexibility and stiffness issues, manufacturers have attempted to use a variety of materials such as vinyl, polyurethane, silicone, natural rubber, polyester and nylon. A drawback with these plastics is they only work well when the wall is sufficiently thick. That is, when the access sheath is constructed with a thin wall made of a plastic or rubber material, the sheath may bend or twist during use. This may result in potential damage as the sharp edge of the kinked sheath may allow an endoscope or other device to complicate the surgical procedure. Moreover, a bent or kinked sheath is useless because it cannot communicate and it does not allow the passage of an instrument. As such, there is a desire in the art for a steerable access sheath that is durable enough to provide sufficient strength and stiffness to be guided through a body cavity or tissue and, at the same time, be flexible enough to perform intricate manipulations through the body cavity or tissue. In particular, it would be desirable to have a steerable access sheath having a thin wall section, a large lumen, an atraumatic distal end and a kink resistant construction.

Wallace (US 2,688,329) describes a catheter operable from its proximal end via a ball knob for effecting deflection of its distal end portion. Mirarchi et al (US 5,865,800) describes a steerable catheter having a distal tip portion deflectable in response to a pull wire within the catheter and an elongated wound wire coil extending through the hollow catheter body of the steerable catheter. Bencini et al (US 6,544,215) describes a steerable device with a handle having a piston that when moved distally a steering wire exerts a pulling force on the distal portion of the elongate body. Biggs (US 6,146,355) describes a steerable catheter with a handle having a steering dial used by an operator to steer a lumen extrusion tip back and forth in one plane. The lumen extrusion tip is fastened to a lumen extrusion shaft that is fastened to a manifold strain relief mounted at the front distal end of the handle. Stevens-Wright et al (EP 0 605 796 A2) describes a steerable electrocardial catheter with an elongated flexible shaft and a flexible tip assembly with a handle/actuator for use with the tip assembly and pull cables for bending the tip assembly via a thumb wheel and a slider enabling four way control of the tip assembly. Webster Jr (US 5,827,278) describes a deflectable tip catheter that includes an elongated catheter body with a deflectable tip section carrying electrodes and a control handle connected to a puller wire fixed to the tip section for reversibly moving the puller wire in a proximal direction to cause deflection of the tip section.

### SUMMARY

According to the present invention there is provided a surgical access device comprising: an elongated body having a proximal end, a distal end, and a steerable region, the body including a primary lumen and a secondary lumen both extending through the body, the secondary lumen having a tensioning device extending through the secondary lumen and connected to the steerable region of the elongated body; and an actuator connected to the tensioning device to control tension of the tensioning device; characterized in further comprising: an enlarged entry at the proximal end of the elongated body, axially aligned with the primary lumen and adapted to receive one of a dilator and an ureteroscope; and a flexible body connected to the proximal end of the elongated body with the tensioning device extending through the flexible body and through the secondary lumen wherein the actuator is connected to the flexible body to situate the actuator remotely and offset from the proximal end of the elongated body.

The steerable portion and the elongate body may have variable stiffness depending on the application of the access sheath. The access sheath has two internal lumen, a primary lumen and a secondary lumen. The primary lumen is sized and configured as an access to a surgical site or the target of a surgical procedure, and operates to advance diagnostic and therapeutic elements to the surgical site or target. The secondary lumen is sized and configured to contain a tensioning device such as a control or pull wire that, when acted upon, will deflect the steerable portion. The tensioning device may be made of a kink resistant material such as Nitinol, a braided cable or any flexible strand or wire.

The access sheath may comprise an extruded multi-lumen plastic tube or a tube molded from a plastic or rubber-like material including polyvinyl chloride, polyester, silicone elastomer, natural or synthetic rubber and polyurethane. The material may range in hardness from around 40 Shore A to 70 Shore D. A structure such as a spring can also be molded into the tube of the access sheath to facilitate kink resistance. More specifically, the access sheath may be formed with an inner plastic body, surrounded by a metal spring coil, which is further covered by an outer plastic body.

In one aspect of steerability of the invention, a tightly wound spring may be placed in the secondary lumen of the access sheath to facilitate movement of the tensioning device inserted therethrough. The spring may be bonded or otherwise fixed to the secondary lumen. Among other features, the spring operates to isolate forces applied by the tensioning device such that only the steerable portion is deflected while the elongate body remains relatively firm when the tensioning device is acted upon. The spring may be coated with a lubricious material further facilitating movement of the tensioning device. The spring may line the entire secondary lumen or portions of the secondary lumen and the spring may be stretched in certain sections to facilitate isolation of the tension force. In another embodiment of the present invention, the tensioning device may be a flattened member extending through at least the steerable portion of the access sheath.

In yet another aspect of the invention, the steerable portion may include a plurality of radially and longitudinally spaced notches and slits disposed on opposite sides of each other facilitating radial deflection of the distal portion in the desired direction or angle. The notches and slits may be of any desired width, length, depth and shape in accordance with the use and flexure requirements of the access sheath. The slits may be narrower and shallower than the notches to provide a "weak-side/strong-side" arrangement of the steerable portion that allows the access sheath to be predisposed to bending in the desired direction.

Many of the attendant features of this invention will be more readily appreciated as the same becomes better understood by reference to the following detailed description and considered in connection with the accompanying drawings in which like reference symbols designate like parts throughout.

### BRIEF DESCRIPTION OF THE DRAWINGS

FIG. 1 illustrates the functioning of a surgical access device or steerable kink resistant access device;
FIG. 2 is a front view of the distal end of the access device of FIG. 1;
FIG. 3 is a rear view of the proximal end of the access device of FIG. 1;
FIG. 4 is an enlarged side view of the distal portion of the access sheath of FIG. 1;
FIG. 5 is a side-section view of the distal portion of the access sheath of FIG. 4;
FIG. 6 illustrates a steerable kink resistant access device with its distal portion deflected;
FIG. 7 is a top view of the distal portion of the access sheath of FIG. 6;
FIG. 8 is a bottom view of the distal portion of the access sheath of FIG. 6;
FIG. 9 illustrates the atraumatic distal end of the access sheath of FIG. 6;

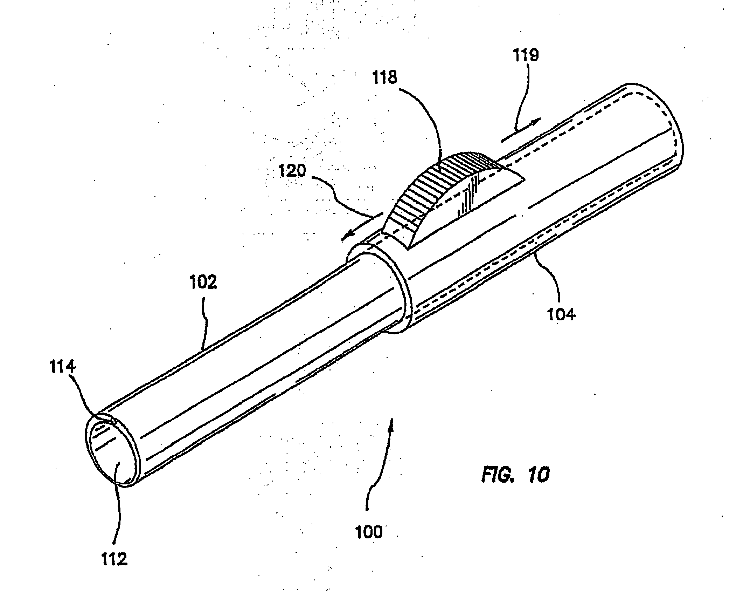

FIG. 10 illustrates an actuator of the access device of FIG. 6 used to control the steerable region or portion of the access sheath;
FIG. 11 illustrates the access device of FIG. 6 guiding a scope into a kidney pole;
FIG. 12 illustrates a perspective view of the distal portion of an access sheath having a flattened tensioning member;
FIG. 13 illustrates a side-elevation view illustrating a spring embodiment of the tube associated with the sheath of the present invention;
FIG. 14 illustrates a perspective view of an actuation hand-piece in accordance with one embodiment of the present invention;
FIG. 15 illustrates a cross-sectional view of a connector in accordance with one embodiment of the present invention;
FIG. 16 illustrates a cross-sectional view of the actuation hand-piece of FIG. 29;
FIGS. 17A-B illustrate perspective views of a disassembled actuation hand-piece in accordance with one embodiment of the present invention;
FIGS. 18A-B illustrate other perspective views of the disassembled actuation hand-piece of FIGS. 17 A-B;
FIG. 19 illustrates a cross-sectional view of the actuation hand-piece of FIG. 32;
FIG. 20 illustrates a perspective view of an actuation hand-piece in accordance with one embodiment of the present invention;
FIGS. 21A-B illustrate cross-sectional views of the actuation hand-piece of FIG. 20;
FIGS. 22-23 illustrate perspective views of embodiments of components of the actuation hand-piece of FIG. 20;
FIG. 24 illustrates a view of an actuation hand-piece in accordance with one embodiment of the present invention;
FIG. 25 illustrates a perspective view of an actuation hand-piece in accordance with one embodiment of the present invention;
FIG. 26 illustrates a perspective view of an actuation hand-piece in accordance with one embodiment of the present invention;
FIG. 27A illustrates a perspective view of an actuation hand-piece in accordance with one embodiment of the present invention;
FIG. 27B illustrates a perspective view of one embodiment of components of the actuation hand-piece of FIG. 27A; and
FIG. 28-31 illustrate cross-sectional views of embodiments of an access sheath in various stages of fabrication in accordance with the present invention.

### DETAILED DESCRIPTION

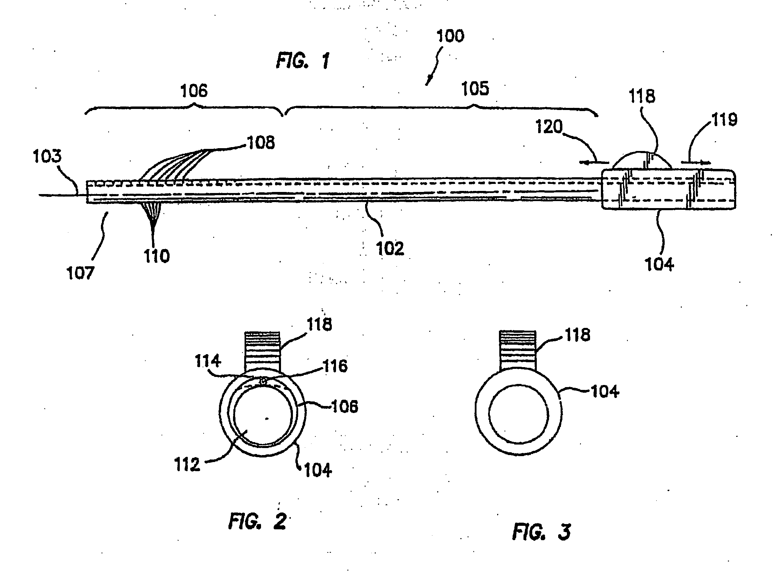

FIGS. 1-3 illustrate a surgical access device or steerable kink resistant access device 100 for use in, among other fields, cardiology, urology, radiology, electrophysiology and gastroenterology. Access device 100 comprises an access sheath 102 having a longitudinal axis 103 extending from a proximal end to a distal end, and a handle portion 104 operatively connected to the proximal end of the access sheath 102. The access sheath 102 includes an elongated body 105 and a steerable region or portion 106. It is appreciated that the steerable portion 106 may be formed anywhere along the access sheath 102. It is further appreciated that the steerable portion 106 and the elongated body 105 may have variable stiffness depending on the application of the access sheath 102. The access sheath 102 has an outside diameter sufficiently small so that it may be inserted into a body cavity or conduit. The access sheath 102 typically has two internal lumen, a primary lumen 112 and a secondary lumen 114, as illustrated in FIG. 2.

The primary lumen 112 is sized and configured as an access to a surgical site or the target of a surgical procedure. In particular, primary lumen 112 operates to advance diagnostic and therapeutic elements to the surgical site or target. The secondary lumen 114 is sized and configured to contain a tensioning device 116 such as a control or pull wire that, when acted upon, will deflect the steerable portion 106 of the access sheath 102. The tensioning device 116 extends through the secondary lumen 114 and is attached to the actuator or handle portion 104 at one end and to a distal portion 107 of the steerable portion 106 at the other end. The handle portion 104 may include a thumb-actuated knob 118 controlling the tensioning device 116. For example, the knob 118 may be drawn proximally in a direction 119 to provide tension to the tensioning device 116 or cause the tensioning device to tense or distally in a direction 120 to loosen tension or cause the tensioning device 116 to loosen.

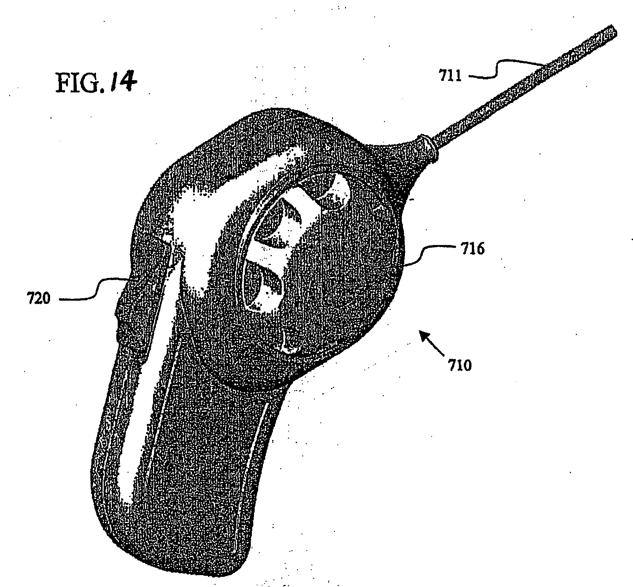

In accordance with the present invention the actuator or actuation hand-piece of the invention is remotely attached to the associated access sheath to control the tensioning and loosening of the tensioning device, for example as described below with reference to Figure 14. In this case, the hand-piece is connected to a flexible tubing or body, which is connected to the access sheath. By providing a remote access point or attachment, the thumbwheels of the hand-piece, for example, may be placed away from the surgical site so that they do not prevent or interfere with full insertion of the working length of the access sheath. It is further appreciated that the access sheath may comprise a plurality of pull wires attached to a plurality of thumbwheels of an actuation hand-piece to deflect the steerable portion of the sheath in different directions.

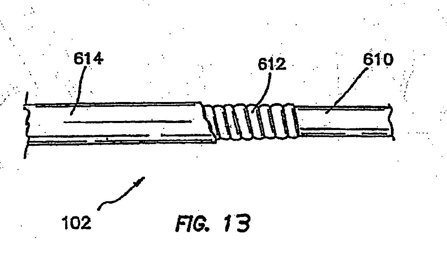

The access sheath 102 in accordance with the invention may comprise an extruded multi-lumen plastic tube. Alternatively, the access sheath 102 may be molded from a plastic or rubber-like material. Preferred materials include polyvinyl chloride, polyester, silicone elastomer, natural or synthetic rubber, polyurethane or the like. The materials may range in hardness from around 40 Shore A to 70 Shore D. These materials are generally flexible and durable. Alternatively, as illustrated in FIG. 13, a structure such as a spring can be molded into the tube of the sheath to facilitate kink resistance. More specifically, the access sheath 102 may be formed with an inner plastic body 610, surrounded by a metal spring coil 612, which is further covered by an outer body 614. This particular embodiment of access sheath 102 provides a high degree of kink resistance. The inner body 610 provides a smooth surface within the sheath, which facilitates passage of instrumentation. The spring coil 612 adds kink resistance to the sheath tube, while the outer body 614 provides a suitable covering for the coils of the spring 612.

In one aspect of steerability of the present invention, a tightly wound spring may be placed in the secondary lumen 114 of the access sheath 102 to facilitate movement of the tensioning device 116 inserted there through. The spring may be bonded or otherwise fixed to the secondary lumen 114. Among other features, the spring operates to isolate forces applied by the tensioning device 116, which is inserted through the spring and is attached to the distal portion 107 of the steerable portion 106. In particular, the spring adds stability and rigidity to the elongate body 105 when the tensioning device 116 is acted upon such that only the steerable portion 106 is bent or steered. Furthermore, the spring operates to direct the tension force applied on the device 116 to the steerable portion 106 so as to allow deflection of only the portion 106 and not the elongate body 105. That is, the tension force is isolated to the steerable portion 106, which may be formed anywhere along the access sheath 102. The spring may be coated with a lubricious material further facilitating movement of the tensioning device 116. The spring may line or cover the inner surface area of the entire secondary lumen 114 or just portions of the secondary lumen 114 to facilitate isolation of the tension force.

The spring may be constructed from a 0.03mm (0.005-inch) diameter wire that is tightly wound forming a closed wound spring having a 0.51 mm (0.02-inch) outer diameter. The distal 13mm to 51 mm (0.5 to 2 inches) of the spring may be stretched to an open wound state such that the windings have an approximately 0.51 mm (0.02-inch) gap between them. This stretched portion of the spring facilitates isolation of the tension force applied by the tensioning device 116. The spring may be coated, for example, in a plastic jacket and bonded to the secondary lumen 114 from the proximal end of the spring to the proximal end of the stretched portion. The stretched portion is then left free to move and/or compress in the plastic jacket. The distal end of the stretched portion may be anchored to the distal end of the access sheath 102 along with the tensioning device 116. The distal end of the plastic jacket may also be bonded to the distal end of the access sheath 102 along with the tensioning device 116 and the spring although these elements do not require a common bonding point or bonding method.

As discussed above, the proximal end of the access sheath 102 is remotely attached to handle portion 104 or actuator or hand-piece 500, which allows the operator to place tension on the tensioning device 116, such as a control or pull wire, while maintaining the position of the catheter. This tension causes the stretched portion of the 0.51mm (0.02-inch) diameter spring to collapse and this, in turn, forces the sheath to bend in the region where the stretched portion of the spring is located. It is appreciated that the stretched portion may be formed anywhere along the catheter or surgical access device that may require bending, and is not limited to the distal end of the device. In addition, more than one deflection assembly of spring and tensioning device may be added to the access device to create deflection in different regions or planes. The amount of bending or deflection will in some way be proportional to the amount of force or tension placed on the tensioning device.

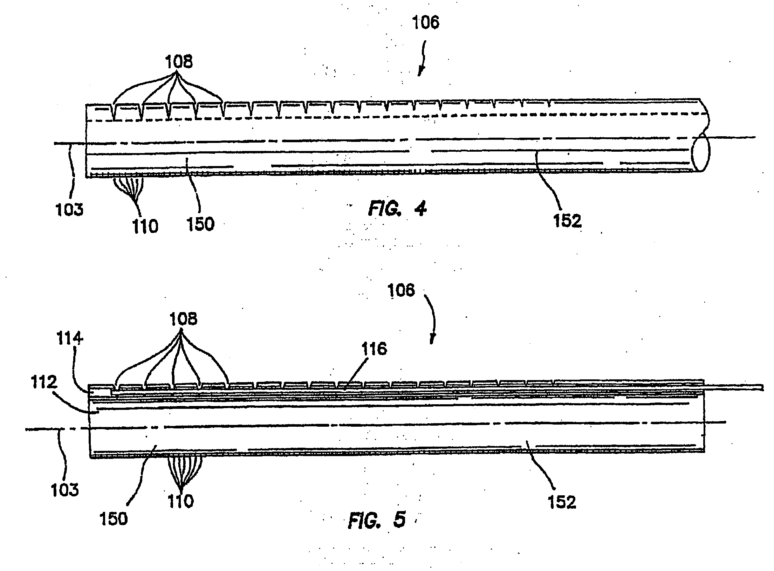

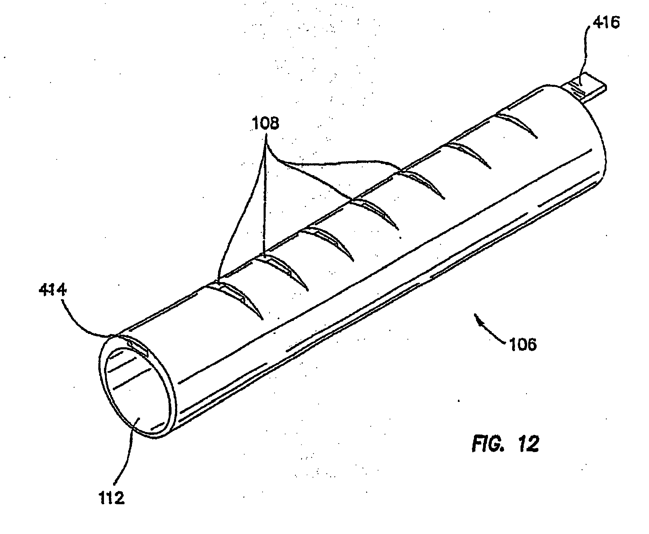

The tensioning device 116 is, in one embodiment, a control or pull wire made of Nitinol, a braided cable or any flexible strand or wire. In one embodiment, the control wire is inserted through the spring such that it runs through the secondary lumen 114 as illustrated in FIG. 5. The proximal end of the tensioning device 116, e.g., a control or pull wire, is connected to an actuator such as the knob 118 of the handle portion 104. The distal end of the control or pull wire, as previously described, is attached to the distal portion 107 of steerable portion 106. In another aspect of the invention as illustrated in FIG. 12, the tensioning device 416 may be a flattened or flat member extending through at least the steerable portion 106 of the access sheath 102.

In another aspect of the present invention as illustrated in FIGS. 1 and 4-5, the steerable portion 106 includes a plurality of radially and longitudinally spaced notches 108 and slits 110 disposed on opposite sides of each other facilitating radial deflection of the distal portion 107 in a desired direction or angle. The notches 108 and slits 110 are cut into the access sheath 102 across the longitudinal axis 103. The degree of deflection may vary greatly based on many factors such as the number, size, direction, shape and spacing of the notches 108 and slits 110. The notches 108 are cut deeper and wider at a distal end 150 than they are at a proximal end 152 of steerable portion 106. The slits 110 comprise of very shallow cuts to provide a reduction in resistance to stretching as the steerable portion 106 is bent or deflected toward the notches 108.

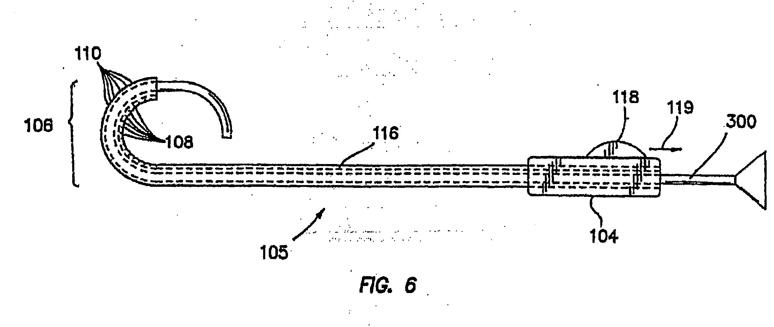

As discussed above, the notches 108 and slits 110 may be of any desired width, length, depth and shape. The number of notches 108 and slits 110 in the steerable portion 106 can be varied in accordance with the use and flexure requirements of the access sheath 102. However, in one embodiment, the slits 110 are narrower and shallower than the notches 108 to provide a "weak-side/strong-side" arrangement of the steerable portion 106 so as to allow the access sheath 102 to be predisposed to bending in the desired direction. That is, when the control wire of the tensioning device 116 is drawn proximally as illustrated in FIG. 6, the more flexible side of the steerable portion 106, i.e., the side with notches 108, will give first thereby bending in the direction of the notches. Moreover, the distal end 150 of the steerable portion 106 with the deeper and wider notches 108 will bend first as the bending progressively moves toward the proximal end 152 having shallower and narrower notches. It is appreciated that the notches 108 may extend through the wall of the access sheath 102.

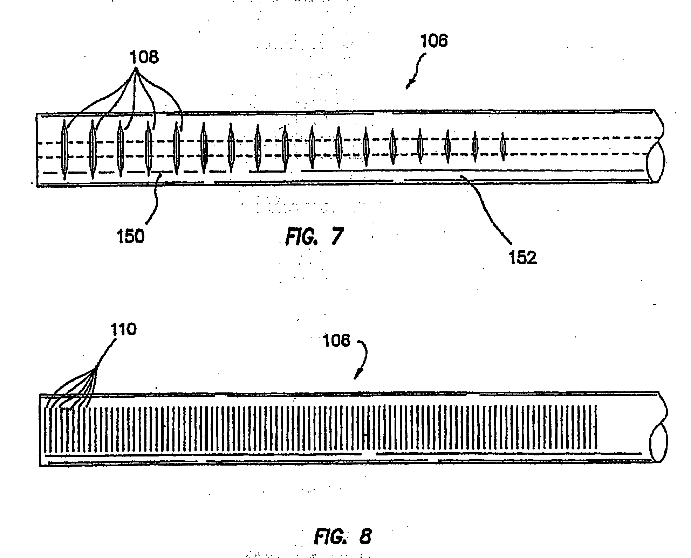

Referring now to FIGS. 7 and 8, the opposing series of notches 108 and slits 110 are further illustrated. The notches 108, as discussed above, provide a "weak-side" or preferred bend path as the notches 108 are closed when bent. It can be seen that the notches 108 are wedge-shaped and have material removed from them. There is, therefore, sufficient room for the material adjacent to each notch to approximate, thereby shortening the length of the steerable portion 106 on the weak-side. In contrast, the slits 110 are shallow radial cuts made directly opposite the notches 108 with little or no material removed. The slits 110 provide the mechanical equivalent of increased plastic elasticity. That is, the slits 110 allow the material of the steerable portion 106 to stretch beyond the intrinsic properties of the material itself. As a result of this construction, the primary lumen 112 of the steerable portion 106 will not collapse when deformed or bent into a tight circular profile as can be seen in FIG. 6. In other words, the slits 110 will only open to provide an elongation of the "strong-side" and will not collapse to provide a shortening of the "strong-side". The material on either side of the notches 108 and slits 110 maintains the general elongate dimension and forms a continuum of the access sheath 102.

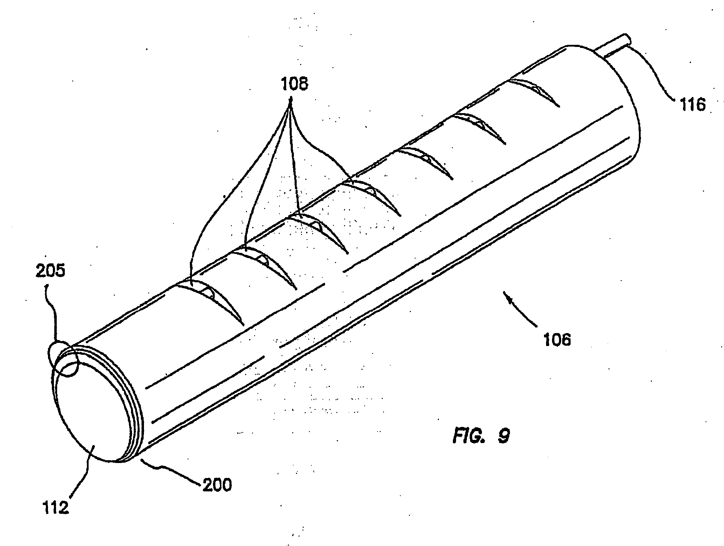

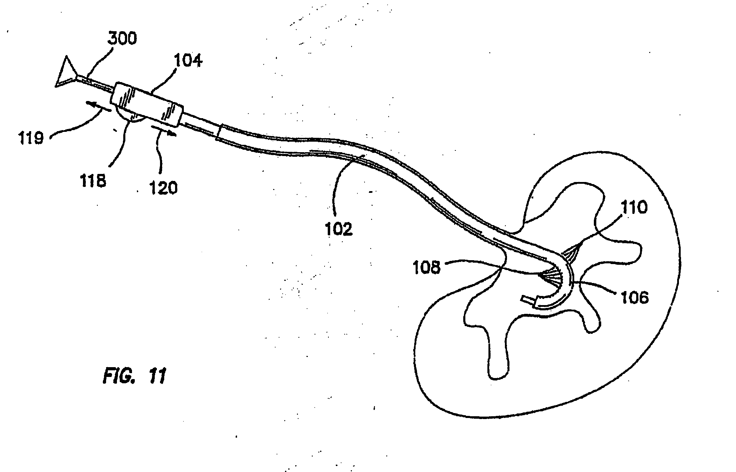

In another embodiment of the invention as illustrated in FIG. 9, the distal end 200 of the steerable portion 106 has a generally rounded off wall section 205 providing an atraumatic insertion tip. With the current construction of the access sheath having a steerable distal portion, less pushing force is required to advance the access sheath since it may be deflected around, under or over anomalies and irregularities in a body cavity or conduit rather than being forced through the tortuous paths. Surgical instruments such as an ureteroscope 300 may be directed through a steerable access sheath as illustrated in FIGS. 6 and 11. For instance, the steerable access sheath may be used to pass the ureteroscope 300 into the upper and lower poles of the renal calices as generally illustrated in FIG. 11. It is appreciated that flexible ureteroscopes and other flexible endoluminal scopes, including completely passive scopes, may be accurately positioned with the assistance of the steerable access sheaths of the present invention.

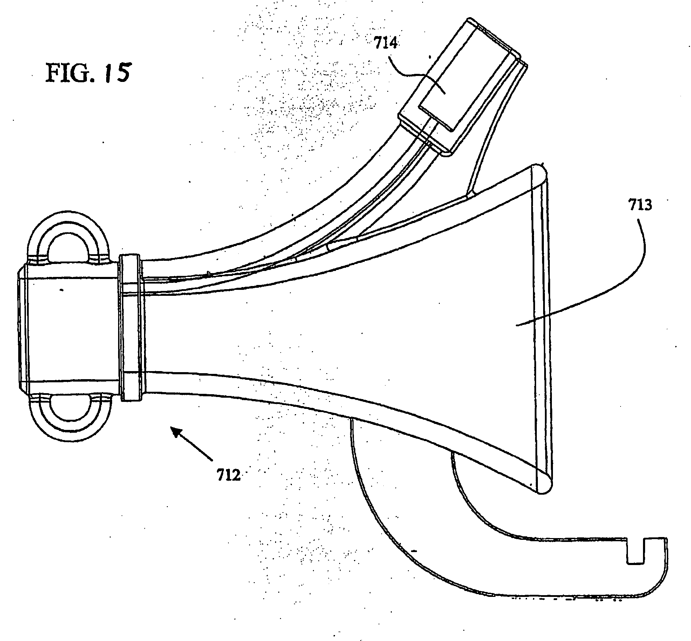

In FIGS. 14-16, one embodiment of an actuator or actuation hand-piece 710 of the present invention is remotely attached to an access sheath to control the tensioning and loosening of a tensioning device connected to the access sheath. As such, the actuator may be placed away from the surgical site or operating path or area so that the hand-piece does not prevent or interfere with the insertion of instruments along the working length of the access sheath. Additionally, the remote actuator does not occupy or add additional working space or length to the access sheath. Furthermore, another user may operate the actuator remotely allowing another user to focus on the surgical procedure, e.g., manipulating instruments to be or already inserted in the access sheath. Extended surgery time and confusion caused by switching between the actuator and other devices or simultaneously using the many devices may be reduced.

The actuator 710, in one embodiment, is connected to a flexible body or conduit 711, which is connected to the access sheath 102 via a Y-connector 712. The Y-connector 712 includes a funnel-shaped entry portion 713 that is sized and arranged to guide instruments into the primary lumen 112 of the access sheath 102. The Y-connector 712 also includes a channel 714 for connecting to the flexible conduit 711. The tensioning device 116 extends through the secondary lumen 114, channel 714, and flexible conduit 711 and is attached to an axle 715 disposed within the actuator 710. The axle 715 is connected to a dial or knob 716 that partially extends laterally from the hand-piece 710 with finger holds disposed radially throughout the knob 716. The other end of the axle 715 is rotatably connected to the hand-piece 710.

The knob 716 allows a user to control the tensioning device 116. For example, when rotated in one direction, e.g., clockwise, the tensioning device 116 is drawn proximally to wrap or wind around the axle 715. A plurality of teeth 717 radially disposed on the knob 716 within the hand-piece 710 or disposed on a separate or embedded ratchet wheel operatively engages with a corresponding lever or pawl 718. The pawl 718 pivoting about a post connected to the hand-piece 710 and biased by a leaf spring 719 engages with the teeth to permit rotational movement of the knob 716 in one direction, e.g., clockwise, while preventing rotational movement in the opposite direction. As such, as the knob 716 is rotated, incremental control of the deflection of the steerable portion 106 of the access sheath 102 is provided as the axle in the hand-piece 710 draws the tensioning device 116 proximally. A trigger 720 when actuated pivots the pawl 718 to disengage the pawl 718 from the teeth 717. As a result, the tensioning device 116 is allowed to unwind or move distally from the axle 715. Thus, the steerable portion 106 of the access sheath 102 straightens.

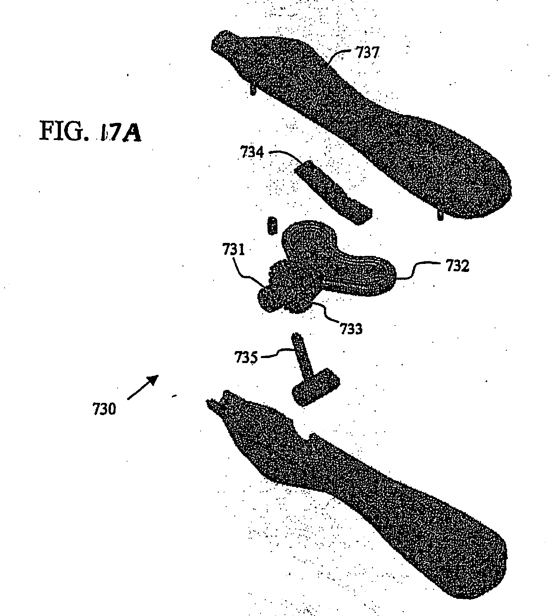

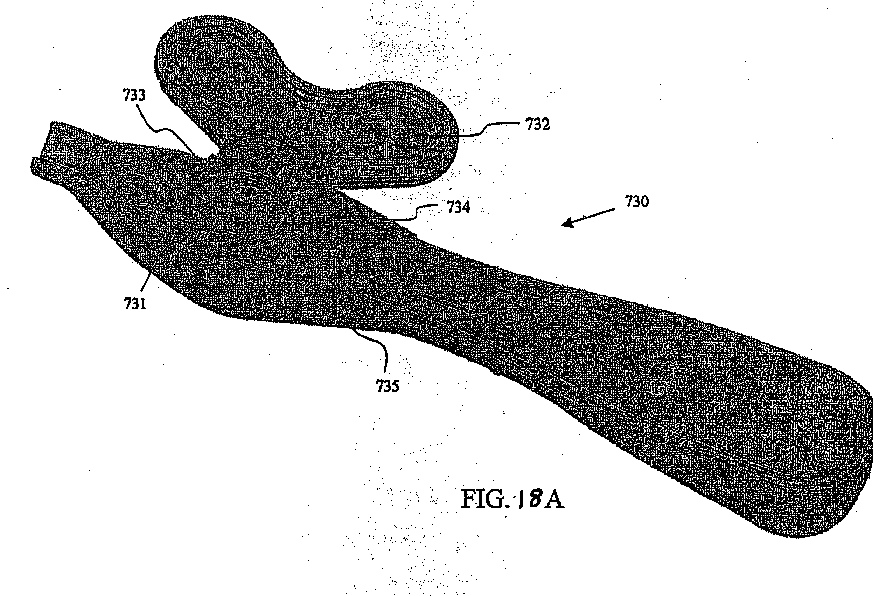

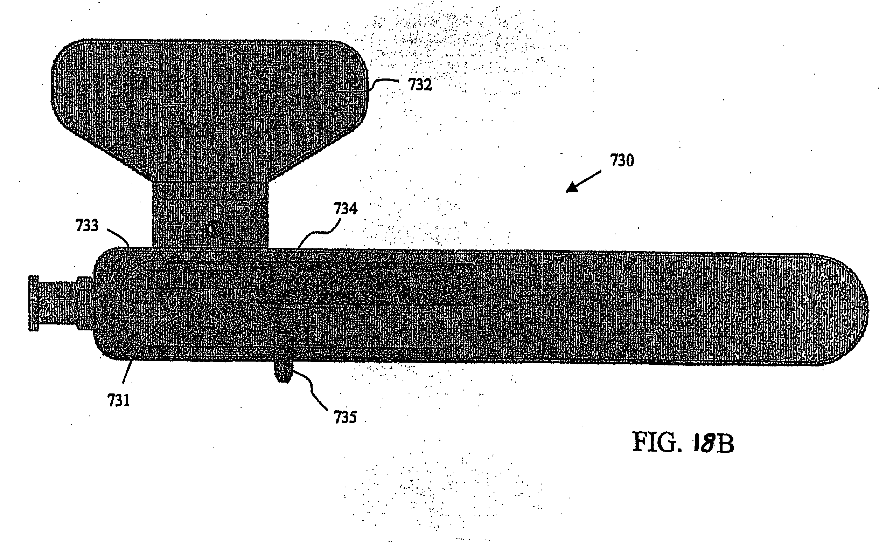

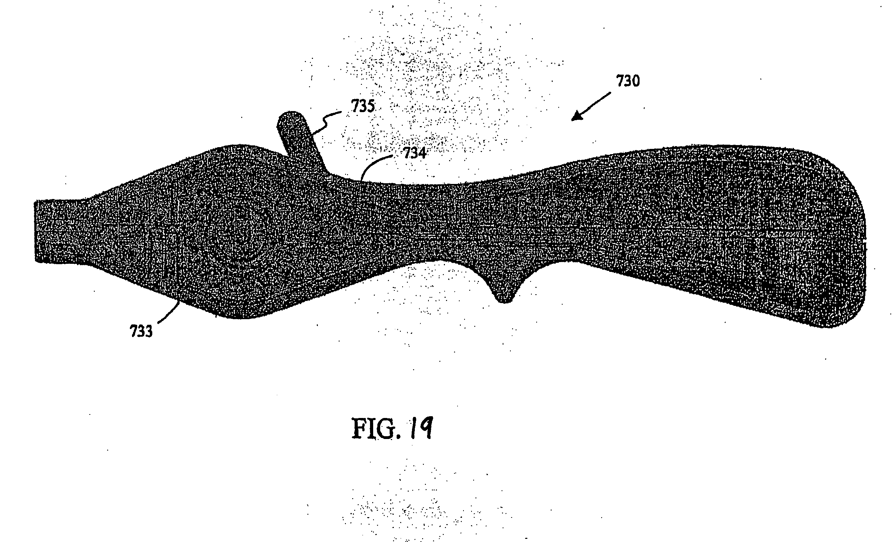

Referring now to FIGS. 17A-19, another embodiment of an actuator or actuation hand-piece 730 of the present invention remotely attached to an access sheath 102 is shown. An axle 731 disposed within the hand-piece 730 is attached to the tensioning device 116. The axle 731 is also connected to a rotatable key or winged lever 732 extending laterally from one side of hand-piece 730.

The lever 732 allows a user to control the tensioning device 116. For instance, when rotated in one direction, e.g., clockwise, the tensioning device 116 is drawn proximally to wrap or wind around the axle 731 in the hand-piece 730. A plurality of teeth 733 radially disposed around the axle 731 or disposed on a ratchet wheel surrounding the axle operatively engages with a corresponding pawl or cantilever arm 734. The arm 734 mounted on the hand-piece 730 and engaged with the teeth 733 permit rotational movement of the lever 732 and axle 731 in one direction while preventing rotational movement in the opposite direction. This engagement provides incremental control of the tensioning device 116 and thus also of the steerable portion 106 of the access sheath 102.

The hand-piece 730 also includes a trigger lever 735 that is pivotally connected to a post in the hand-piece 730 and partially extends through a slot in the hand-piece 730. The lever 735 when actuated, e.g., pulled proximally, moves the cantilever arm 734 to disengage from the teeth 733 to allow the axle 731 to freely rotate. Therefore, the tensioning device 116 connected to the axle 731 unwinds and/or moves distally from the axle 731 causing the steerable portion 106 of the access sheath 102 to straighten.

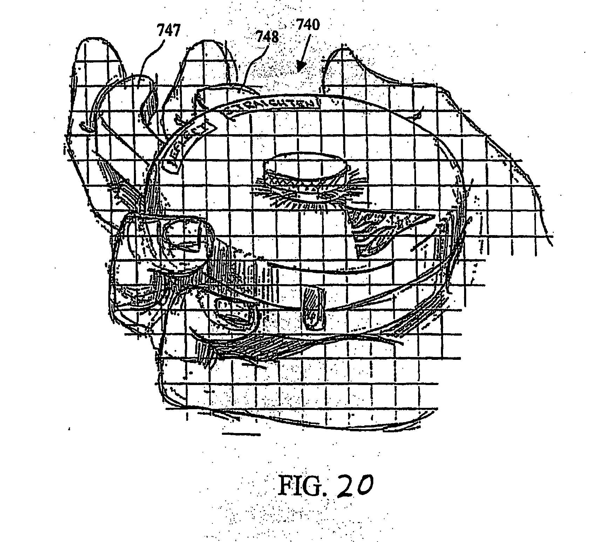

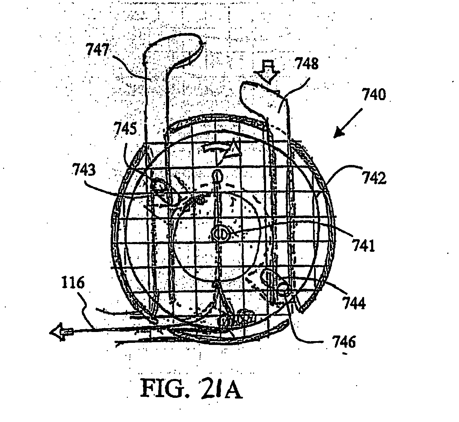

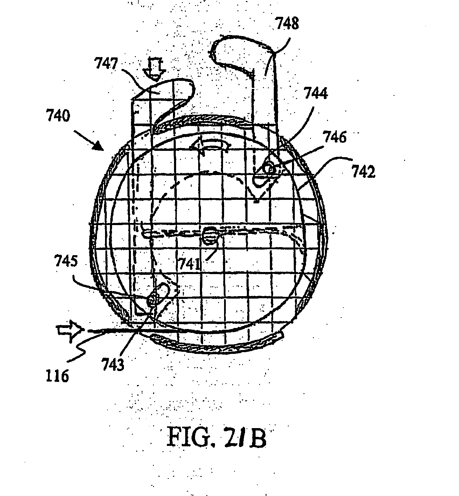

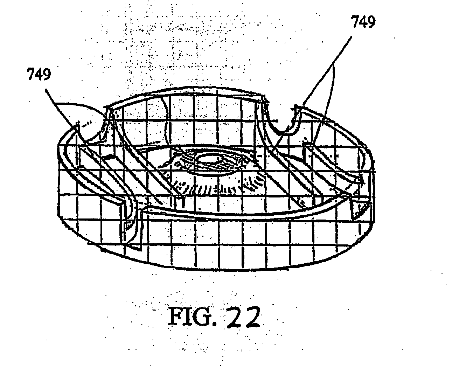

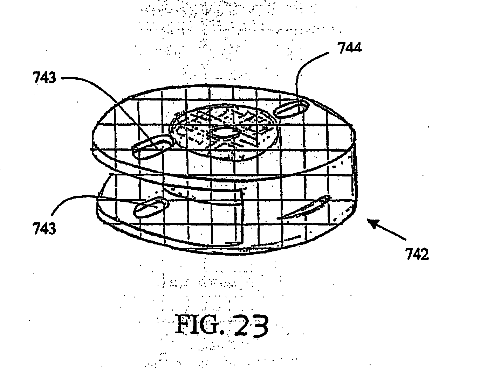

In FIGS. 20-23, another embodiment of an actuator or actuation hand-piece 740 of the present invention that is remotely attached to an access sheath 102 to control the tensioning and loosening of the tensioning device 116 is shown. Disposed within the hand-piece 740 is an axle 741, which is attached to tensioning device 116. The axle 741 is connected to a slotted wheel 742 that is partially rotatable within the hand-piece 740. Generally opposing slots 743 and 744 are disposed in the slotted wheel through which respective dowels or pins 745 and 746 extend through and connect to distal ends of respective button arms 747 and 748. Proximal ends of button arms 747 and 748 extend through openings in the hand-piece 740.

Operationally, when the button arm 748 is lowered, the button arm 747 rises as the slotted wheel 742 rotates clockwise. As a result, the tensioning device 116 connected to axle 741 is not drawn to the hand-piece 740, such that the steerable portion 106 of the access sheath 102 is substantially straight. When button arm 747 is lowered, the button arm 748 rises and the slotted wheel 742 rotates causing the tensioning device 116 to be pulled by rotating axle 741 such that the steerable portion 106 of access sheath 102 deflects. In one aspect of the present invention, the hand-piece 740 includes guides 749 for aligning and guiding traversal of the button arms 747 and 748 and slots (not shown) for assisting linear movement of the pins 745 and 746 as the button arms move. The slotted wheel 742 also includes one or more openings along the circumference of the wheel to permit rotation of the wheel without interfering with the guides 749. In another aspect of the present invention, the axle 741 is connected to a screw knob for adjusting the tension or pre-winding the tensioning device 116 around the axle 741.

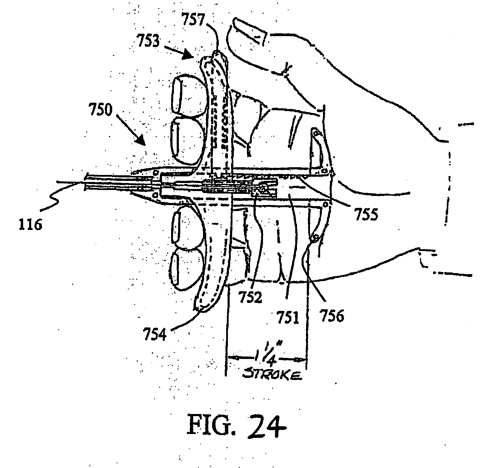

Referring now to FIG. 24, another embodiment of a remotely attached actuator or actuation hand-piece 750 is shown. In one aspect of the present invention, the hand-piece 750 fits within a user's hand in that a fist closing motion moves a t-bar 753 proximally deflecting the access sheath 102 and an opening motion moves the t-bar 753 distally allowing the access sheath 102 to straighten. For example, the hand-piece 750 includes finger-extension members 754 to provide one or more fingers on each member 754 to grasp the t-bar 753. A distally flared end 756 of a tube 751 is also provided for resting in the palm of a hand.

Extending through a distal end of the tube 751 is the tensioning device 116 that attaches to plate 752 within the tube 751. The plate 752 is connected to the t-bar 753 that is slidably connected to tube 751. In one embodiment, an adjustment screw is connected to the t-bar 753 to adjust the location of the plate 752 relative to the t-bar 753 and within the tube 751. A set of teeth 755 within tube 751 operatively engages with a tooth or detent on t-bar 753 as the t-bar 753 moves.

With the t-bar 753 moving proximally, plate 751 also moves proximally thereby pulling tensioning device 116 to cause the steerable portion 106 of the access sheath 102 to deflect. Similarly, as the t-bar 753 and plate 751 moves distally, the tensioning device 116 loosens and thus the steerable portion 106 straightens. As such, this engagement provides incremental control of the deflection and/or straightening of the steerable portion 106 of the access sheath 102. A spring-loaded button 757 on one end of the t-bar 753, when actuated, disengages the tooth on t-bar 753 from the teeth 755 within tube 751 allowing the t-bar 753 to move freely.

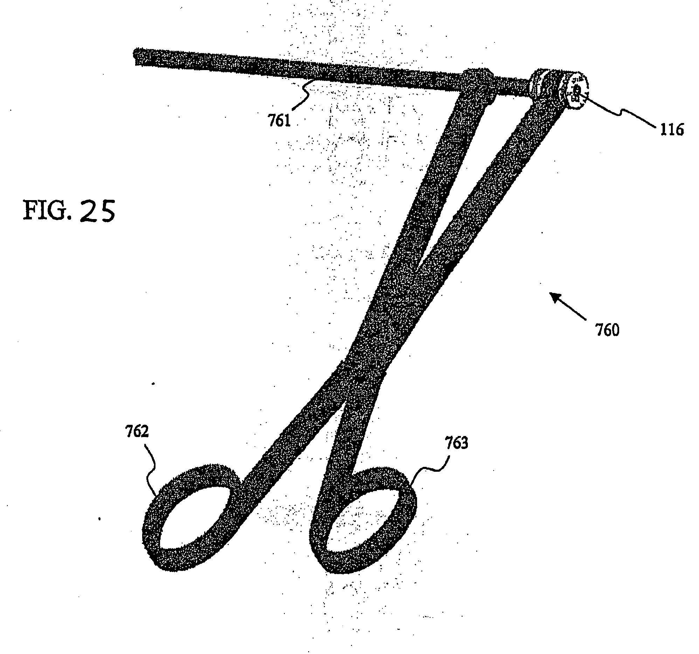

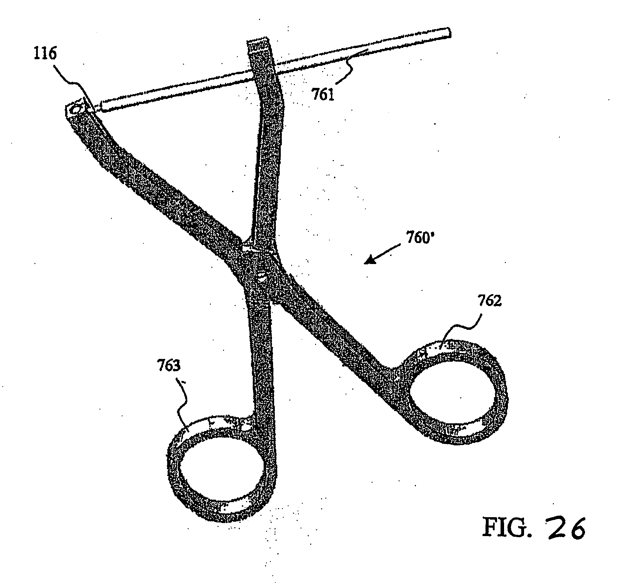

FIGS. 25-26 illustrate another embodiment of the present invention of an actuator or actuation hand-piece 760 and 760' each remotely attachable to the access sheath 102. The hand-piece 760 and 760' are connected to a flexible body or tube 761 through which the tensioning device 116 extends. The tensioning device 116 is attached to a first handle member 762 that is pivotally connected to a second handle member 763. Actuation of the first handle member 762 allows a user to pull or release the tensioning device 116 to respectively deflect or straighten the steerable portion 106 of the access sheath 102. In one aspect of the present invention, a ratchet assembly is included to provide incremental control of the tensioning device 116 and thus the deflection of the steerable portion 106 of the access sheath 102.

It is appreciated that the access sheath may comprise a plurality of pull wires attached to a plurality of thumbwheels, axles, knobs or other types of movable components of an actuation hand-piece to deflect the steerable portion of the sheath in different directions. Also, it is appreciated that the tensioning device may be hydraulic, pneumatic or electronic in nature and the actuation hand-piece may instead be foot, finger or otherwise sensor actuated and may include corresponding foot, finger or otherwise sensor extensions.

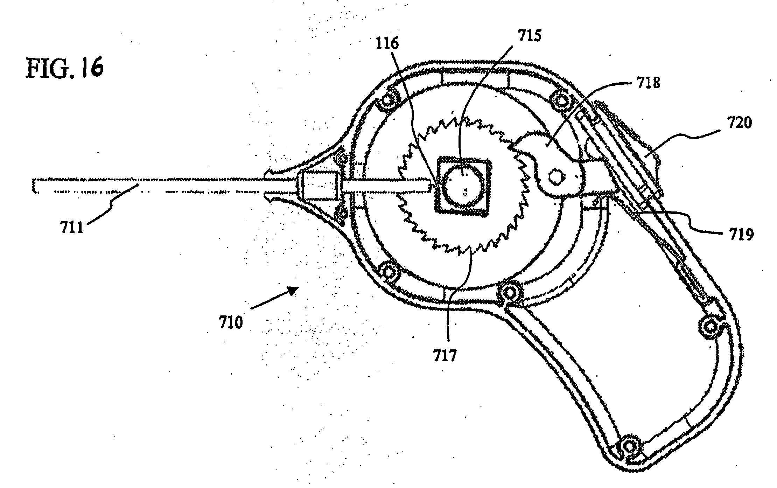

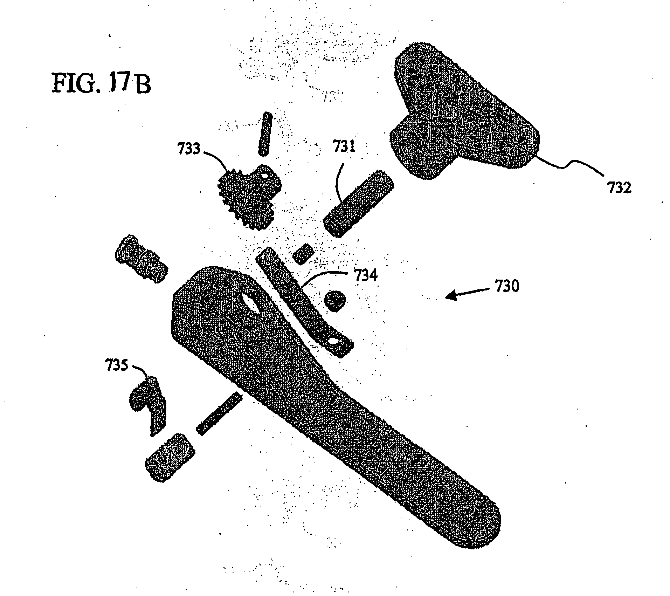

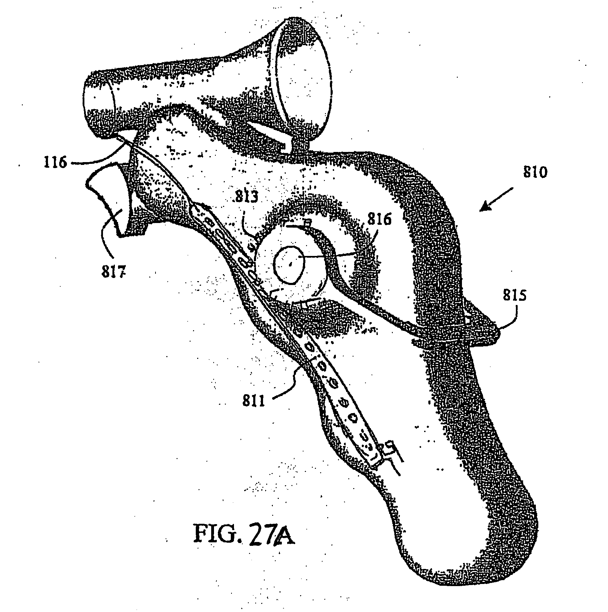

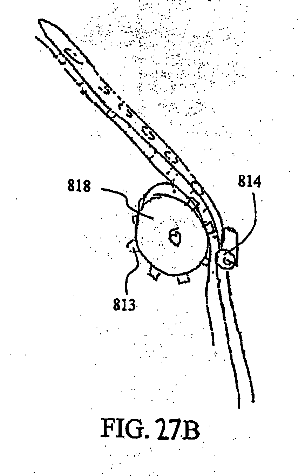

In various embodiments, for example, the embodiments previously described and/or the embodiment of an actuation hand-piece 810 shown in FIGS. 27A-B, the tensioning device 116 is connected to a belt 811. The belt 811 acts as an intermediary between the tensioning device 116 and a movable component 818 in the hand-piece 810. The movable component may also be, for example, slider, cylinder, movable handle member, axle 715 or 731 (FIGS. 16 and 17), and various other movable components fully or partially disposed within or otherwise part of an actuator or hand-piece which is connectable to the tensioning device 116. Through belt 811, stress or forces that may be applied by or result from the movable component 818 is displaced from the tensioning device 116. Therefore, stress experienced by the tensioning device 116 caused by the actuation of the hand-piece may be reduced.

The belt 811 includes a number of apertures 812 for engaging teeth 813 radially disposed on the movable component 818 of the hand-piece 810. In one embodiment, the belt 811 includes teeth or protrusions for engaging corresponding apertures, teeth or protrusions of the movable component 818. With either engagement, incremental control of the tensioning device 116 is provided. As such, the belt 811 draws the tensioning device 116 proximally as the knob is rotated in one direction and rotating the knob in the opposite direction, allows the tensioning device to withdraw from the hand-piece 810.

A pin or roller 814 may also be included to assist in the engagement of the belt 811 with a movable component 818 of the hand-piece 810. In one aspect of the present invention, the belt 811 is pliable. In another embodiment, a plate, bar, or a less flexible component may be connected to the belt 811 for drawing or releasing the belt 811 in conjunction with or without the movable component 818.

A u-shaped lever 815 is connected to a knob 816 that is disposed on one or both sides of the hand-piece 810 and is connected to the movable component 818 in the hand-piece 810. Through actuation of the u-shaped lever 815, a user can control the movement/tension of the tensioning device 116 and thus the deflection and straightening of the steerable portion 106 of the access sheath 102. In one embodiment, a plate is connected to the u-shaped lever 815 and the belt 811 to draw and release the tensioning device 116.

In one aspect of the present invention, a trigger 817, when actuated, locks the belt 811, the movable component 818 or the u-shaped lever 815, thus preventing further movement of the tensioning device 116 and the deflection/straightening of the steerable portion 106 of the access sheath 102. Alternatively, the trigger 817 releases or disengages control of the tensioning device 116 from the belt 811, the movable component 818 or the u-shaped lever 815 to allow the tensioning device 116 to return to its original position or state.

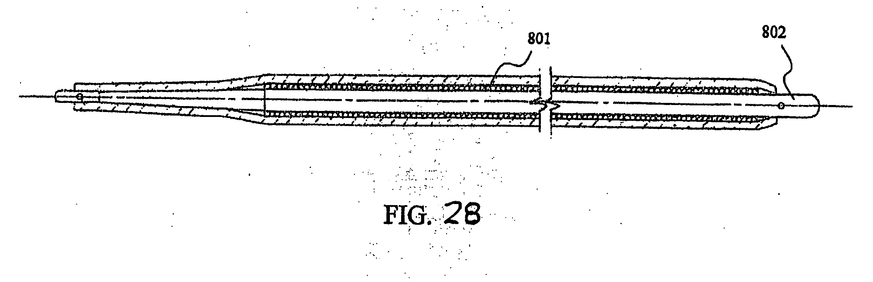

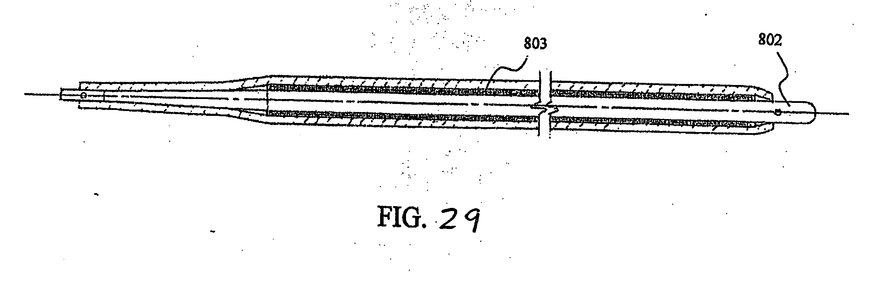

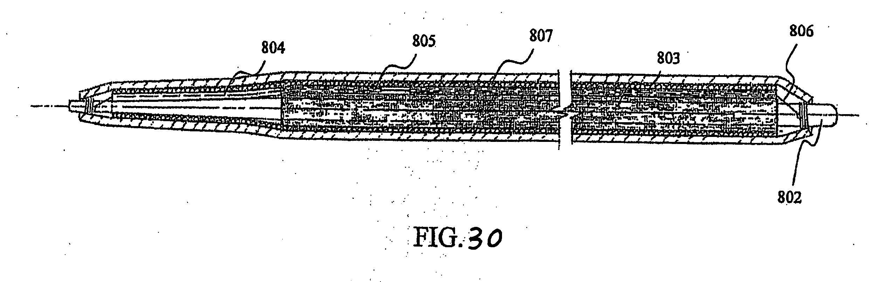

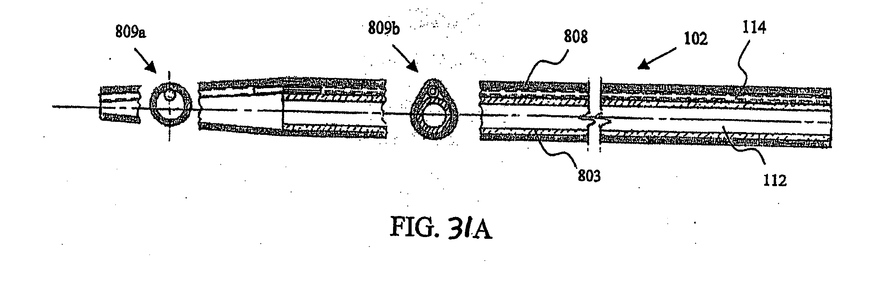

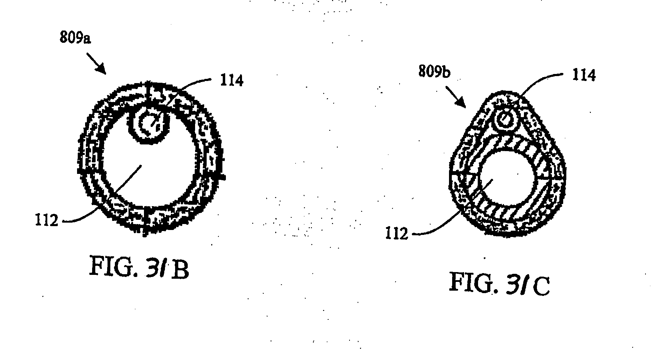

Referring now to FIGS. 28-32, embodiments of an access sheath in various stages of fabrication is shown, which may be used in an access device of the present invention. A wire 801 is wound around a support member or mandrel 802 in which the size and shape of mandrel generally defines the size and shape of primary lumen 112 of the access sheath 102. The mandrel, in one embodiment, is stainless steel and made of or is coated with a low friction material or surface, e.g., Teflon or various mold releases, allowing for the mandrel to be easily removed from the access sheath 102. The wire 801 is wound in an over counter fashion by using anchors or starting and stopping points substantially orthogonal of each other and thus winding the wire 801 in an oblique line along mandrel 802. As such, the wire 801 is wound such that the wire's tendency to unwind is counteracted. In one embodiment, prior to the addition of the wire 801, the mandrel 802 is coated with or inserted into a plastic or PVC material tube to allow instruments and the like to be smoothly inserted into the primary lumen without interference from the wire 801.

The wire 801, in one embodiment, is a plastic coated wire and particularly, a stainless steel co-extruded wire with an approximate diameter of .006 inches fused, coated or otherwise included with a plastic material to make the total diameter of the wire 801 to be about 0.3mm (.012 inches). The mandrel 802 including wire 801 is placed into or inserted into a control tube. Air, in one embodiment, is supplied, e.g., at 690KPa (100 PSI), on the opposite end of insertion to assist insertion of the mandrel 802 by expanding the control tube. The control tube, in one embodiment, may be made of silicon or a material with a higher melting point than the plastic coating of wire 801. This assembly is then heated such that the plastic coating of wire 801 melts and adheres to itself to form a generally continuous tubular structure or major tube 803. The control tube is then removed.

A minor tube 804 is placed on or included with the major tube 803. The minor tube 804 is longer than the major tube 803 and thus extends substantially further along the mandrel 802 than the major tube 803. Extending within a portion of the minor tube 804 is a generally tubular structure or inner tube 805 that is about as long as the major tube 803. In one embodiment, the inner tube 805 is made of polyimide and the minor tube 804 is made of carbothane that when heated adheres to the inner tube 805, the major tube 803 and other portions of the access sheath, which are described below, that surrounds the outer periphery of the minor tube 804.

The inner tube 805 within the minor tube 804 is adapted to receive the support wire 806. The size and shape of the support wire 806 along with the inner tube 805 generally defines the size and shape of the secondary lumen 114 of the access sheath 102. In one embodiment, the support wire is a stainless steel wire with a diameter of about .12 inches. The support wire 806 is secured to a proximal end of the mandrel 802, threaded through the inner tube 805 and the minor tube 804 and secured to the distal end of the mandrel 802. In one embodiment, the support wire 806 secures the minor tube 804 to the major tube 803.

The minor tube 804 extends along the mandrel 802 substantially more than the inner tube 805. In other words, the length of the minor tube 804 is longer than the inner tube 805. The minor tube 804 is also more flexible than the inner tube 805. As such, the portion from the end point of the inner tube 805 and/or the major tube 803 to near the end point of the minor tube 804 eventually defines the steerable portion 106 of the access sheath 102. In one embodiment, the minor tube 804 is shorter and less flexible than the inner tube 805. Thus, in this embodiment, the portion from the end point of the minor tube 804 and/or the major tube 803 to near the end point of the inner tube 805 eventually defines the steerable portion 106 of the access sheath 102.

In one embodiment, the minor tube 804, inner tube 805 and the major tube 803 are placed into a final tube to enclose the minor tube 804 and inner tube 805 between the major tube 803 and the final tube. This assembly is placed into or inserted into a control tube such that the assembly adheres or bonds together and then the control tube is removed.

In one embodiment, the minor tube 804 or the inner tube 805, whichever extends further, is rigid, e.g., a stainless steel tube, to assist in the deflection of the steerable region 106. As such, the rigidity of the minor tube 804 or inner tube 804 prevents the non-steerable portion of the access sheath 102 from bowing. As such, the tube shifts the force caused by the tensioning device 116 to deflect the steerable region directly towards or at the steerable region 106. Also, a rigid secondary lumen formed by the rigid tube may assist in the protection of the tensioning device and instruments inserted or withdrawn from the primary lumen.

A wire 807 is wound around the minor tube 804, the inner tube 805 and the major tube 803. In one embodiment, where the final tube is utilized, the wire 807 is also wound around the final tube. In one embodiment, the wire 807 is similar in construction or composition as that of wire 801 and/or extends slightly beyond the distal end of the minor tube 804 or inner tube 805.

A support tip, in one embodiment, is placed on a distal end or slightly beyond the distal end of the wire 807 to assist in securing the wire 807 around the minor tube 804 or inner tube 805 and/or to provide an atraumatic tip. The support tip may be a 75 Shore D material. The mandrel 802 with rest of the assembly is inserted into a control tube. As previously mentioned, air, in one embodiment, is supplied on the opposite end of insertion to assist insertion of the mandrel 802 by expanding the control tube. In one aspect, a support tube is used to temporarily encompass the control tube when the tube is pressurized in the event the tube breaks down. The control tube with the assembly is heated such that the plastic coating of wire 807 melts and adheres to itself to form a generally continuous tubular structure or tube 808. The control tube is then removed. In one embodiment, the control tube and assembly are heated at around 165 degrees plus or minus about five to ten degrees for about ten to fifteen minutes. As such, an access sheath 102 with a variable flexibility is created.

The support wire 806 is disconnected from the mandrel 802. For example, the support wire 806 on the distal end of the mandrel 802 is cut and then the mandrel 802 is withdrawn from the access sheath 803. At or near the tip of the access sheath, a tensioning device, e.g., a pull wire, is attached and threaded to the minor tube 804 and inner tube 805 out the proximal end of the access sheath 102 for securing to an actuator. As such, the access sheath is deflectable and controllable.

In one embodiment, the tensioning device is knotted or looped around an opening or cut in the access sheath, the support tip and/or between loops in the wire 807 and back through itself. A catch wire threaded through the inner tube 805 and the minor tube 804 hooks or otherwise attaches to the tensioning device. The catch wire is removed out the proximal end of the access sheath thereby threading the tensioning device through and out the proximal end of the access sheath 102. As it is appreciated the support wire 806 has a diameter sufficiently larger than the diameter of the tensioning device, the catch wire or loops and hooks of the catch wire to permit easy passage of these devices through the secondary lumen of the access sheath 102. A secondary support tip, in one embodiment, is placed on the distal end of the access sheath 102 to assist in securing the tensioning device to the access sheath and/or to provide an atraumatic tip.

As shown in FIGS. 46A-C, the distal end 809a of the access sheath 102 is tapered and thus has a smaller diameter than the proximal end 809b of the access sheath 803. The primary lumen 112 and secondary lumen 114 diameters, however, remain substantially constant throughout the access sheath 102. Additionally, the tapering or reduced diameter of the access sheath is a result of the halting or non-extension of the inner tube 805 or minor tube 804, in one embodiment, and the major tube 803 along the length of the mandrel 802. As a result, the steerable portion 106 includes a reduced amount of materials and more flexible materials, and thus the steerable portion is easily deflected, bent, shaped or curved in response to the manipulation of the attached tensioning device while the other portion of the access sheath 102, including more material and less flexible material, remains substantially fixed, e.g., straight and substantially in the same plane, preventing any inadvertent or unintended movement of the access sheath.

Additionally, since the steerable region 106 of the access sheath 102 is reinforced by wire 807, the steerable region 106 is strengthen such that a flexible, pre-bendable or otherwise not actively controllable instrument may be controllably deflected dynamically as the steerable region 106 is controlled. Additionally, an actively deflectable surgical instrument may have a complicated construction providing components, e.g., optics or clamps, to perform its surgical function and components to perform the active deflection. Therefore, such instruments may be fragile or if broken may be expensive to replace or repair or still usable as a surgical instrument but not actively deflectable. As such, the strengthen steerable region 106 may replace the components or use of the components in such surgical instruments or induce an broken instrument to be controllably deflected thereby reducing replacement, repair and/or construction costs, reducing wear and tear of such instruments and increasing the life of such instruments. Also, the reinforced access sheath 102 through wire 807 and/or wire 801 allows the size and shape of the primary lumen to remain substantially constant throughout the access sheath 102, thereby reducing forces on instruments placed within the access sheath which may extend the life of these instruments.

The forces or stress accumulated along the access sheath that may cause kinks in the access sheath are also distributed along the access sheath due to the composite construction of the access sheath described above and are further counteracted by the wire coils, e.g., wire 807 and 803. Thus, kinks in the access sheath are reduced. The wire coils also allow the access sheath walls to be very thin without reducing durability or strength in the access sheath. Thus, the overall or outer diameter of the access sheath may be small, which may also reduce the incision or insertion point for the access sheath, without reducing the size or diameter of the primary lumen. As such, the access sheath of various embodiments of the present invention has thin walled portions, a large lumen, an atraumatic end, and a kink resistant construction and is strong, stiff and yet flexible enough to be intricately guided through the body cavity or tissue. In one embodiment, the wire coils are wound in a multifilar fashion with materials having alternating durometers.

Various other examples of processes that may be used to manufacture the access sheath 102 or portions of the access sheath 102 are described in U.S. Patent Application Nos. 10/766,138 and 10/298,116,. It is appreciated that these processes or portions of the processes may be varied or combined with the previously described process and vice versa. For example, various ring-shaped elements, such as, plastic rings, metallic rings, un-reinforced plastic rings and metal reinforced plastic rings, and the like may be utilized instead of or in addition to the wires 803 and/or 807. Additionally, a separate mandrel may be utilized to separately form or define the primary and secondary lumens and combined to make the access sheath.

In one embodiment of the present invention, various embodiments of access sheaths and actuators previously described, here now referred to as the access sheath, combined with an instrument or device used to stretch or enlarge an opening, e.g., a dilator, allows for gradual and atraumatic dilation of the ureter while being placed. Once the access sheath has been placed at a desired location, the dilator is removed and the access sheath is left in place. The access sheath allows for continued access to the desired area, for example, for the placement of an ureteroscope and other therapeutic instruments, while providing protection of the ureter. For instance, the access sheath may protect the ureter during the placement and removal of devices within the access sheath, during the removal of stone fragments or other tissue, and during the removal of a potentially cancerous biopsy specimen.

Additionally, with the access sheath being deflectable or steerable, an urologist may effectively and efficiently locate stones and stone fragments within the kidney. When a stone burden is found in one of the calyces of the kidney, especially in the lower pole portion of the kidney; it may be difficult for the urologist to continue to go back to the same calyx or location to remove the burden.

When there are many fragments within a calyx, many entries and exits may be performed to remove the burden. Also, when a stone or stone fragment is removed, the instruments and tissue, e.g., the scope and stone basket (with the stone or stone fragment) are removed as a single unit. The scope is then passed back through the sheath and manipulated to find the same calyx in order to remove the remaining burden. However, with the access sheath 102, the access sheath can be left deflected in place looking at the same calyx or location, while the scope and stone basket are removed. As a result, the urologist's procedure time may be reduced, as the urologist may not have to manipulate the ureteroscope to look for the same calyx each time. The amount of time saved may be significant, especially if there is a large stone burden within the kidney. Additionally, the likelihood of doing damage to the kidney due to the additional manipulation that takes place every time the ureteroscope is placed back into the kidney may be reduced.

Thus, with the access sheath, one can keep the sheath deflected towards a particular calyx and remove the stone burden without having to find the calyx each and every time a fragment is removed.

When the urologist manipulates an ureteroscope, the urologist may sometimes use the inside walls of the kidney to help deflect the ureteroscope to enter into a particular difficult locale. With the access sheath 102, instead of using the inside wall to help deflect the ureteroscope the access sheath may be used. Also, as previously mentioned, this will also help reduce the "wear and tear" on surgical instruments, such as ureteroscopes. The deflecting mechanism with the ureteroscope, if provided, can be damaged often and expensive repair. The use of the access sheath may reduce the damage to the ureteroscope when it is used to help manipulate the ureteroscope to desired locations within the kidney.

The use of the access sheath 102 may also help a lesser-experienced urologist perform the same difficult procedure as their more experienced colleagues. In performing this procedure, the urologist may access the lower pole of the kidney in order to remove a stone burden. By performing this procedure in a retrograde fashion, one can reduce a patient's recovery time. If an urologist were neither skilled nor comfortable with using an ureteroscope in a retrograde fashion to remove a stone burden from a kidney's lower pole, the urologist would typically approach the stone burden in an antegrade fashion. This places a sheath percutaneously and thus may add additional recovery time for a patient as well as potentially increasing morbidity. But, with the access sheath 102 and an ureteroscope, an urologist may efficiently and effectively locate and remove a stone burden within the lower pole of a kidney. The access sheath can also be used in an antegrade fashion and will provide the same or similar features described above, however access in this manner may not be the preferred method.

Although this invention has been described in certain specific embodiments, many additional modifications and variations would be apparent to those skilled in the art which fall within the scope of the invention as claimed.

## Claims

1. A surgical access device comprising:
an elongated body (102) having a proximal end, a distal end, and a steerable region (106), the body including a primary lumen (112) and a secondary lumen (114) both extending through the body, the secondary lumen having a tensioning device (116)_extending through the secondary lumen and connected to the steerable region (106) of the elongated body(102); and
an actuator (710) connected to the tensioning device (116) to control tension of the tensioning device (116); **characterized in** further comprising:
an enlarged entry (713) at the proximal end of the elongated body, axially aligned with the primary lumen (112) and adapted to receive one of a dilator and an ureteroscope; and
a flexible body (711) connected to the proximal end of the elongated body with the tensioning device extending through the flexible body and through the secondary lumen (114)
wherein the actuator (710) is connected to the flexible body (711) to situate the actuator remotely and offset from the proximal end of the elongated body (102).

2. The access device of claim 1 wherein the tensioning device (116) is at least one pull wire.

3. The access device of claim 1 wherein the tensioning device (416) is a flexible flatten member.

4. The access device of claim 1 wherein the enlarged entry (713) is funnel-shaped.

5. The access device of claim 1 wherein the secondary lumen (114) has a diameter smaller than a diameter of the primary lumen (112).

6. The access device of claim 1 wherein the actuator further comprises one of a thumbwheel (508, 632, 633), a knob (716, 816), a lever (732, 735, 815), a button (747, 748, 757), a handle (622, 762, 763), a t-bar (753), and a dial (533, 534, 716).

7. The access device of claim 6 wherein the actuator further comprises a hand engaging extension (754).

8. The access device of claim 7 wherein the actuator further comprises a directional indicator (512).

9. The access device of claim 1 wherein the actuator further comprises means for incrementally controlling tension of the tensioning device (716, 717, 718).

10. The access device of claim 1 wherein the actuator further comprises a movable component (715) disposed within the actuator (710) and connected to the tensioning device (116).

11. The access device of claim 10 wherein the actuator comprises:
a plurality of teeth (717, 733) connected to the movable component (715); and
a pawl (718) operatively engaged with the first set of teeth (717).

12. The access device of claim 11 wherein the actuator (810) further comprises an extension member (811) connected between the tensioning device (116) and the movable component (818).

13. The access device of claim 12 wherein the extension member (811) operatively engages the plurality of teeth (813).

14. The access device of claim 10 wherein the actuator comprises:
a first set of teeth (717, 755) connected to the movable component;
a second set of teeth (733) operatively engaged with the first set of teeth;
a pawl (718, 734) operatively engaged with the first set of teeth; and
a trigger (720) extending from the actuator and operatively engaged with the pawl (718, 734).

15. The access device of claim 1 wherein the actuator in a first position pulls the tensioning device (116) and in a second position releases the tensioning device (116).

16. The access device of claim 1 further comprising a spring fixed within the secondary lumen (114) and wherein the tensioning device (116) extends through the spring.

17. The access device of claim 1 wherein the body further comprises at least one other secondary lumen (114) disposed around the primary lumen (112).

18. The access device of claim 1 wherein the body comprises an inner plastic body (610), a spring coil (612) and an outer plastic body (614).

19. The access device of claim 18 wherein the inner plastic body (610) is surrounded by the spring coil (612) and covered by the outer plastic body (614).

20. The access device of claim 1 wherein the steerable region (106) includes a weakened portion extending along the steerable region (106).

21. The access device of claim 1 wherein the steerable region (106) includes slots (108) extending along the steerable region (106) and slits (110) extending along the steerable region (106) on a generally opposing side of the slots (1080, the slots (108) being larger than the slits (110).

22. The access device of claim 1 wherein the steerable region includes means for increasing elasticity of the steerable region (106).

23. The access device of claim 1 wherein the tube is adapted to operate in a kidney.

24. The access device of claim 1 wherein the steerable region (106) has a length adapted to use in a calyx.

25. The access device of claim 1 further comprises means for incrementally controlling the tensioning device (716, 717, 718) and coupled to the actuator (710,810).

26. The access device of claim 1 wherein the elongate body being:
a tube (102) having a substantially rigid portion having a first diameter and a substantially flexible portion having a second diameter and extending from the substantially rigid portion, the first diameter being smaller than the second diameter, the tensioning device (116) being a pull wire extending through the secondary lumen (114) and connected to the flexible portion of the elongated body;
a connector (712) having a distal end connected to the tube and a proximal end including a funnel-shaped portion (713), the pull wire extending through the connector (712) from the distal end to the proximal end;
the flexible body (711) being a plastic tubing connected to the connector (713) through which the pull wire extends through; and
the actuator (710, 810) being a handle (622, 762, 763) connected to the plastic tubing and including an axle (715, 731) disposed within the handle (622, 762, 763) and a knob (716, 816) connected to and outside the handle (622, 762, 763), the axle (715, 731) connected to the pull wire and the knob (716, 816).

27. The access device of claim 26 further comprises:
a pawl (718, 734) connected to the handle (622, 762, 763);
a trigger (720) connected to the handle (622, 762, 763) and the pawl (718, 734); and
a first set of teeth (717, 755) connected to the axle (715, 731) and operatively engaging with the pawl (718, 734);
wherein the trigger (720) when actuated causes the pawl (718, 734) to move to disengage the pawl (718, 734) from the first set of teeth (717, 755).

28. The access device of claim 27 further comprising an extension member connected between the axle (715, 731) and the pull wire and having a second set of teeth (733) and operatively engaging the first set of teeth (717, 755).

## Patentansprüche

1. Chirurgisches Zugangsgerät, das Folgendes umfasst:
ein länglicher Körper (102) mit einem proximalen Ende, einem distalen Ende und einem lenkbaren Bereich (106), wobei das Körper ein Primärlumen (112) und ein Sekundärlumen (114) umfasst, die sich beide durch den Körper erstrecken, wobei das Sekundärlumen eine Spannvorrichtung (116) hat, die sich durch das Sekundärlumen erstreckt und am lenkbaren Bereich (106) des länglichen Körpers (102) angeschlossen ist, und
ein Stellglied (710), das an der Spannvorrichtung (116) zur Kontrolle der Spannung der Spannvorrichtung (116) angeschlossen ist, **dadurch gekennzeichnet, dass** es ferner Folgendes umfasst:
einen erweiterten Zugang (713) am proximalen Ende des länglichen Körpers, der axial mit dem Primärlumen (112) ausgerichtet und so adaptiert ist, dass er einen Dilatator und ein Urethroskop erhält, und
ein flexibler Körper (711), der am proximalen Ende des länglichen Körpers angeschlossen ist, wobei sich die Spannvorrichtung durch den flexiblen Körper und durch das Sekundärlumen (114) erstreckt,
wobei das Stellglied (710) am flexiblen Körper (711) angeschlossen ist, um das Stellglied vom proximalen Ende des länglichen Körpers (102) ferngesteuert und versetzt zu platzieren.

2. Zugangsgerät nach Anspruch 1, wobei die Spannvorrichtung (116) mindestens ein Drahtzug ist.

3. Zugangsgerät nach Anspruch 1, wobei die Spannvorrichtung (416) ein flexibles abgeflachtes Element ist.

4. Zugangsgerät nach Anspruch 1, wobei der erweiterte Zugang (713) trichterförmig ist.

5. Zugangsgerät nach Anspruch 1, wobei das Sekundärlumen (114) einen Durchmesser hat, der kleiner als ein Durchmesser des Primärlumens (112) ist.

6. Zugangsgerät nach Anspruch 1, wobei das Stellglied ferner ein Daumenrad (508, 632, 633), einen Knopf (716, 816), einen Hebel (732, 735, 815), eine Taste (747, 748, 757), einen Griff (622, 762, 763), eine T-Stange (753) und eine Skala (533, 534, 716) umfasst.

7. Zugangsgerät nach Anspruch 6, wobei das Stellglied ferner eine in Eingriff stehende Verlängerung umfasst (754).

8. Zugangsgerät nach Anspruch 7, wobei das Stellglied ferner einen direktionalen Indikator (512) umfasst.

9. Zugangsgerät nach Anspruch 1, wobei das Stellglied ferner ein Mittel zur inkrementellen Kontrolle der Spannung der Spannvorrichtung (716, 717, 718) umfasst.

10. Zugangsgerät nach Anspruch 1, wobei das Stellglied ferner eine bewegliche Komponente (715) umfasst, die innerhalb des Stellglieds (710) angeordnet und an der Spannvorrichtung (116) angeschlossen ist.

11. Zugangsgerät nach Anspruch 10, wobei das Stellglied Folgendes umfasst:
eine Vielzahl einer Verzahnung (717, 733), die an der beweglichen Komponente (715) angeschlossen sind, und
eine Klinke (718), die mit der ersten Verzahnung (717) funktionsfähig im Eingriff steht.

12. Zugangsgerät nach Anspruch 11, wobei das Stellglied (810) ferner ein Verlängerungselement (811) umfasst, das zwischen der Spannvorrichtung (116) und der beweglichen Komponente (818) angeschlossen ist.

13. Zugangsgerät nach Anspruch 12, wobei das Verlängerungselement (811) funktionsfähig mit der Vielzahl der Verzahnung (813) im Eingriff steht.

14. Zugangsgerät nach Anspruch 10, wobei das Stellglied Folgendes umfasst:
eine erste Verzahnung (717, 755), die an der beweglichen Komponente angeschlossen ist.
eine zweite Verzahnung (733), die mit der ersten Verzahnung funktionsfähig im Eingriff steht,
eine Klinke (718, 734), die mit der ersten Verzahnung funktionsfähig im Eingriff steht, und
einen Auslöser (720), der sich vom Stellglied erstreckt und mit der Klinke (718, 734) funktionsfähig im Eingriff steht.

15. Zugangsgerät nach Anspruch 1, wobei das Stellglied in einer ersten Position die Spannvorrichtung (116) zieht und in einer zweiten Position die Spannvorrichtung (116) löst.

16. Zugangsgerät nach Anspruch 1, das ferner eine Feder umfasst, die innerhalb des Sekundärlumens (114) befestigt ist, und wobei sich die Spannvorrichtung (116) durch die Feder erstreckt.

17. Zugangsgerät nach Anspruch 1, wobei der Körper ferner mindestens ein anderes Sekundärlumen (114) umfasst, das um das Primärlumen (112) herum angeordnet ist.

18. Zugangsgerät nach Anspruch 1, wobei der Körper einen inneren Kunststoffkörper (610), eine Sprungfeder (612) und einen äußeren Kunststoffkörper (614) umfasst.

19. Zugangsgerät nach Anspruch 18, wobei der innere Kunststoffkörper (610) von der Spiralfeder (612) umgeben ist, und von einem äußeren Kunststoffkörper (614) abgedeckt wird.

20. Zugangsgerät nach Anspruch 1, wobei der lenkbare Bereich (106) einen abgeschwächten Abschnitt beinhaltet, der sich am lenkbaren Bereich (106) entlang erstreckt.

21. Zugangsgerät nach Anspruch 1, wobei der lenkbare Bereich (106) Spalten (108) beinhaltet, die sich am lenkbaren Bereich (106) entlang erstrecken, und Schlitze (110) die sich am lenkbaren Bereich (106) entlang an einer allgemein gegenüberliegenden Seite der Spalten (108) erstrecken, wobei die Spalten (108) größer als die Schlitze (110) sind.

22. Zugangsgerät nach Anspruch 1, wobei der lenkbare Bereich ein Mittel zur Erhöhung der Elastizität des lenkbaren Bereichs (106) beinhaltet.

23. Zugangsgerät nach Anspruch 1, wobei die Röhre so adaptiert wurde, dass sie in einer Niere funktioniert.

24. Zugangsgerät nach Anspruch 1, wobei der lenkbare Bereich (106) eine Länge hat, die zur Verwendung in einem Nierenkelch adaptiert wurde.

25. Zugangsgerät nach Anspruch 1, das ferner ein Mittel zur inkrementellen Kontrolle der Spannung der Spannvorrichtung (716, 717, 718) umfasst und am Stellglied (710, 810) angekoppelt ist.

26. Zugangsgerät nach Anspruch 1, wobei der längliche Körper Folgendes umfasst:
eine Röhre (102), die im Wesentlichen einen starren Abschnitt hat, der einen ersten Durchmesser und einen im Wesentlichen flexiblen Abschnitt hat, mit einem zweiten Durchmesser, der sich vom im Wesentlichen starren Abschnitt erstreckt, wobei der erste Durchmesser kleiner als der zweite Durchmesser ist, und die Spannvorrichtung (116) ein Drahtzug ist, der sich durch das Sekundärlumen (114) erstreckt und am flexiblen Abschnitt des länglichen Körpers angeschlossen ist,
ein Verbindungsstück (712), das ein distales Ende hat, das an der Röhre angeschlossen ist, und ein proximales Ende, das einen trichterförmigen Abschnitt (713) beinhaltet, wobei sich der Drahtzug durch das Verbindungsteil (712) vom distalen Ende zum proximalen Ende erstreckt,
wobei der flexible Körper (711) ein Kunststoffrohr ist, das am Verbindungsteil (713) angeschlossen ist, durch das sich der Drahtzug erstreckt, und
das Stellglied (710, 810), das ein Griff (622, 762, 763) ist, der am Kunststoffrohr angeschlossen ist und eine Achse (715, 731) beinhaltet, die innerhalb des Griffs (622, 762, 763) angeordnet ist, und eine Taste (716, 816), die außerhalb des Griffs (622, 762, 763) angeschlossen ist, wobei die Achse (715, 731) am Drahtzug und an der Taste (716, 816) angeschlossen ist.

27. Zugangsgerät nach Anspruch 26, das außerdem Folgendes umfasst:
eine Klinke (718, 734), die am Griff (622, 762, 763) angeschlossen ist,
einen Auslöser (720), der am Griff (622, 762, 763) und an der Klinke angeschlossen ist (718, 734) und
eine erste Verzahnung (717, 755), die an der Achse (715, 731) angeschlossen ist und operativ mit der Klinke (718, 734) im Eingriff steht,
wobei der Auslöser (720), wenn er aktiviert ist, verursacht, dass sich die Klinke (718, 734) bewegt, um die Klinke (718, 734) von der ersten Verzahnung (717, 755) zu lösen.

28. Zugangsgerät nach Anspruch 27, das ferner ein Verlängerungsteil umfasst, das zwischen der Achse (715, 731) und dem Drahtzug angeschlossen ist, und eine zweite Verzahnung (733) hat, und operativ mit der ersten Verzahnung (717, 755) im Eingriff steht.

## Revendications

1. Dispositif d'accès chirurgical comprenant :
un corps allongé (102) ayant une extrémité proximale, une extrémité distale et un segment dirigeable (106), le corps incluant une lumière primaire (112) et une lumière secondaire (114), s'étendant toutes deux dans le corps, la lumière secondaire ayant un dispositif de tension (116) s'étendant dans la lumière secondaire et étant relié au segment dirigeable (106) du corps allongé (102) ; et
un actionneur (710) relié au dispositif de tension (116) servant à contrôler la tension du dispositif de tension (116) ; **caractérisé, en outre, en ce qu'**il comprend :
une entrée élargie (713) à l'extrémité proximale du corps allongé, alignée axialement avec la lumière primaire (112) et adaptée de manière à recevoir un dilatateur et un urétéroscope ; et
un corps souple (711) relié à l'extrémité proximale du corps allongé, le dispositif de tension s'étendant dans le corps souple et dans la lumière secondaire (114),
dans lequel l'actionneur (710) est relié au corps souple (711) pour situer l'actionneur à distance et le décaler par rapport à l'extrémité proximale du corps allongé (102).

2. Dispositif d'accès selon la revendication 1, dans lequel le dispositif de tension (116) est au moins un fil de traction.

3. Dispositif d'accès selon la revendication 1, dans lequel le dispositif de tension (416) est un membre aplati souple.

4. Dispositif d'accès selon la revendication 1, dans lequel l'entrée élargie (713) est en forme d'entonnoir.

5. Dispositif d'accès selon la revendication 1, dans lequel la lumière secondaire (114) a un diamètre inférieur au diamètre de la lumière primaire (112).

6. Appareil Dispositif d'accès selon la revendication 1, dans lequel l'actionneur comprend, en outre, l'un des éléments suivants : une molette (508, 632, 633), une olive (716, 816), un levier (732, 735, 815), un bouton (747, 748, 757), une poignée (622, 762, 763), une barre en T (753), et un cadran (533, 534, 716).

7. Dispositif d'accès selon la revendication 6, dans lequel l'actionneur comprend, en outre, une rallonge à main (754).

8. Dispositif d'accès selon la revendication 7, dans lequel l'actionneur comprend, en outre, un indicateur directionnel (512).

9. Dispositif d'accès selon la revendication 1, dans lequel l'actionneur comprend, en outre, un moyen servant à contrôler la tension du dispositif de tension (716, 717, 718) par paliers.

10. Dispositif d'accès selon la revendication 1, dans lequel l'actionneur comprend, en outre, un composant mobile (715) disposé à l'intérieure de l'actionneur (710) et relié au dispositif de tension (116).

11. Dispositif d'accès selon la revendication 10, dans lequel l'actionneur comprend :
une pluralité de dents (717, 733) reliées au composant mobile (715) ; et
un cliquet (718) s'engageant activement avec le premier ensemble de dents (717).

12. Dispositif d'accès selon la revendication 11, dans lequel l'actionneur (810) comprend, en outre, un membre rallonge (811) connecté entre le dispositif de tension (116) et le composant mobile (818).

13. Dispositif d'accès selon la revendication 12, dans lequel le membre rallonge (811) s'engage activement avec la pluralité de dents (813).

14. Dispositif d'accès selon la revendication 10, dans lequel l'actionneur comprend :
un premier ensemble de dents (717, 755) relié au composant mobile ;
un second ensemble de dents (733) activement engagé avec le premier ensemble de dents ;
un cliquet (718, 734) activement engagé avec le premier ensemble de dents ; et
un déclencheur (720) s'étendant entre l'actionneur et étant activement engagé avec le cliquet (718, 734).

15. Dispositif d'accès selon la revendication 1, dans lequel l'actionneur, dans une première position, tire le dispositif de tension (116) et, dans une seconde position, libère le dispositif de tension (116).

16. Dispositif d'accès selon la revendication 1 comprenant, en outre, un ressort fixé à l'intérieur de la lumière secondaire (114) et dans lequel le dispositif de tension (116) s'étend dans le ressort.

17. Dispositif d'accès selon la revendication 1, dans lequel le corps comprend, en outre, au moins une autre lumière secondaire (114), disposée autour de la lumière primaire (112).

18. Dispositif d'accès selon la revendication 1, dans lequel le corps comprend un corps en matière plastique interne (610), un ressort en spirale (612) et un corps en matière plastique externe (614).

19. Dispositif d'accès selon la revendication 18, dans lequel le corps en matière plastique interne (610) est entouré par le ressort en spirale (612) et recouvert par le corps en matière plastique externe (614).

20. Dispositif d'accès selon la revendication 1, dans lequel le segment dirigeable (106) inclut une partie affaiblie s'étendant le long du segment dirigeable (106).

21. Dispositif d'accès selon la revendication 1, dans lequel le segment dirigeable (106) inclut des fentes (108) s'étendant le long du segment dirigeable (106) et des rainures (110) s'étendant le long du segment dirigeable (106) sur un côté, en général, opposé aux fentes (108), les fentes (108) étant plus larges que les rainures (110).

22. Dispositif d'accès selon la revendication 1, dans lequel le segment dirigeable inclut un moyen pour augmenter l'élasticité du segment dirigeable (106).

23. Dispositif d'accès selon la revendication 1, dans lequel le tube est adapté de manière à opérer dans un rein.

24. Dispositif d'accès selon la revendication 1, dans lequel le segment dirigeable (106) a une longueur adaptée pour l'utilisation dans une couronne dentée.

25. Dispositif d'accès selon la revendication 1, comprenant, en outre, un moyen pour contrôler le dispositif de tension par paliers (716, 717, 718) et le coupler à l'actionneur (710, 810).

26. Dispositif d'accès selon la revendication 1, dans lequel le corps allongé comprend :
un tube (102) ayant un segment substantiellement rigide ayant un premier diamètre et un segment substantiellement souple ayant un second diamètre et s'étendant à partir du segment substantiellement rigide, le premier diamètre étant plus petit que le second diamètre, le dispositif de tension (116) possédant un fil de traction s'étendant dans la lumière secondaire (114) et relié au segment souple du corps allongé ;
un raccord (712) ayant une extrémité distale reliée au tube et une extrémité proximale incluant une partie en forme d'entonnoir (713), le fil de traction s'étendant dans le raccord (712) entre l'extrémité distale et l'extrémité proximale ;
le corps souple (711) étant un tube en matière plastique relié au raccord (713) dans lequel le fil de traction s'étend ; et
l'actionneur (710, 810) étant une poignée (622, 762, 763) reliée au tube en matière plastique et incluant un axe (715, 731) disposé à l'intérieur de la poignée (622, 762, 763) et une olive (716, 816) reliée à et située hors de la poignée (622, 762, 763), l'axe (715, 731) étant connecté au fil de traction et à l'olive (716, 816).

27. Dispositif d'accès selon la revendication 26 comprenant, en outre,
un cliquet (718, 734) relié à la poignée (622, 762, 763) ;
un déclencheur (720) relié à la poignée (622, 762, 763) et le cliquet (718, 734) ;
un premier ensemble de dents (717, 755) relié à l'axe (715, 731) et engagé activement avec le cliquet (718, 734) ;
dans lequel le déclencheur (720), lorsqu'il est actionné, cause le déplacement du cliquet (718, 734) en vue de dégager le cliquet (718, 734) du premier ensemble de dents (717, 755).

28. Dispositif d'accès selon la revendication 27 comprenant, en outre, un membre rallonge relié entre l'axe (715, 731) et le fil de traction et ayant un second ensemble de dents (733) et s'engageant activement avec le premier ensemble de dents (717, 755).
